# EUROPEAN PATENT APPLICATION

(11) **EP 3 098 603 A1**
(43) Date of publication of application: **30.11.2016**
(21) Application number: 16170333.5
(22) Date of filing: 19.05.2016
(51) Int. Cl.: G01N 33/543, G01N 33/553, G01N 33/58, G01N 33/66

(54) **GEL FOR SENSOR AND SENSOR**

(30) Priority: 21.05.2015 JP 2015103839
(71) Applicant: Seiko Epson Corporation, Shinjuku-ku Tokyo 163-0811 (JP)
(72) Inventor: YOSHIOKA, Satomi, Nagano, 392-8502 (JP); YAGI, Hiroshi, Nagano, 392-8502 (JP)
(74) Representative: Hoffmann Eitle

(57) **Abstract**

A sensor includes a gel for a sensor and an electrode. The gel for a sensor includes a stimulus-responsive gel, first particles which are contained in the stimulus-responsive gel and are constituted by a material containing an electrically conductive substance, and second particles which are contained in the stimulus-responsive gel and contribute to structural coloration. It is preferred that when the electrical resistivity of the electrically conductive substance is represented by R1 (Ω·m) and the electrical resistivity of the constituent material of the second particles is represented by R2 (Ω·m), the following relationship is satisfied: 1×10¹² ≤ R2/R1 ≤ 1×10²³. It is preferred that when the refractive index of the constituent material of the first particles is represented by n1 and the refractive index of the constituent material of the second particles is represented by n2, the following relationship is satisfied: -0.8 ≤ n2-n1 ≤ 1.5.

## Description

### BACKGROUND

### 1. Technical Field

The present invention relates to a gel for a sensor and a sensor.

### 2. Related Art

At present, as a method for obtaining in vivo biological information, a biochemical test in which the composition of the blood obtained by blood collection is examined is generally performed. This test is mostly performed in medical institutions. Above all, a blood glucose sensor has been widely used in diabetic patients, and also a simple lactic acid sensor is getting widely used in athletes.

However, both are test methods involving blood collection using an invasive technique.

On the other hand, as a method using a non-invasive technique, a sensor targeting a component of sweat has been studied (see, for example, Wearable Technology for Bio-Chemical Analysis of Body Fluids During Exercise 30th Annual International IEEE EMBS Conference Vancouver, British Columbia, Canada, August 20-24, 2008, and Novel lactate and pH biosensor for skin and sweat analysis based on single walled carbon nanotubes/Sensors and Actuators B 117 (2006) 308-313).

However, an enzyme used in such a method is generally expensive and is susceptible to temperature, humidity, etc., and therefore has problems that stable properties are hard to exhibit and the reliability of quantitativeness is low. In addition, the enzyme greatly varies in quality among production lots or manufacturers, and also has a problem that its properties change greatly over time.

### SUMMARY

An advantage of some aspects of the invention is to provide a sensor capable of easily and stably performing detection of the strength of a stimulus (the concentration or the like of a given component) in a wide range, and also to provide a gel for a sensor which can be favorably used for a sensor capable of easily and stably performing detection of the strength of a stimulus (the concentration or the like of a given component) in a wide range.

A gel for a sensor according to an aspect of the invention includes a stimulus-responsive gel, first particles which are contained in the stimulus-responsive gel and are constituted by a material containing an electrically conductive substance, and second particles which are contained in the stimulus-responsive gel and contribute to structural coloration.

According to this configuration, a gel for a sensor which can be favorably used for a sensor capable of easily and stably performing detection of the strength of a stimulus (the concentration or the like of a given component) in a wide range can be provided.

In the gel for a sensor according to the aspect of the invention, it is preferred that when the electrical resistivity of the electrically conductive substance is represented by R1 (Ω·m) and the electrical resistivity of the constituent material of the second particles is represented by R2 (Ω·m) , the following relationship is satisfied: 1×10¹² ≤ R2/R1 ≤ 1×10²³.

In the gel for a sensor according to the aspect of the invention, it is preferred that the electrical resistivity of the electrically conductive substance is 1.0×10⁻³ Ω·m or less.

In the gel for a sensor according to the aspect of the invention, it is preferred that the electrically conductive substance is constituted by a material containing one or more members selected from the group consisting of a metal material, an electrically conductive metal oxide, a carbon material, and an electrically conductive polymer material.

In the gel for a sensor according to the aspect of the invention, it is preferred that when the refractive index of the constituent material of the first particles is represented by n1 and the refractive index of the constituent material of the second particles is represented by n2, the following relationship is satisfied: -0.8 ≤ n2-n1 ≤ 1.5.

In the gel for a sensor according to the aspect of the invention, it is preferred that the average particle diameter of the second particles is 50 nm or more and 1000 nm or less.

In the gel for a sensor according to the aspect of the invention, it is preferred that the content of the first particles with respect to 100 parts by volume of the stimulus-responsive gel when the stimulus-responsive gel is in an expanded state is 0.1 parts by volume or more and 50 parts by volume or less.

In the gel for a sensor according to the aspect of the invention, it is preferred that the content of the second particles with respect to 100 parts by volume of the stimulus-responsive gel when the stimulus-responsive gel is in an expanded state is 0.1 parts by volume or more and 50 parts by volume or less.

In the gel for a sensor according to the aspect of the invention, it is preferred that the gel for a sensor is provided with a recessed portion in a region coming into contact with an electrode.

In the gel for a sensor according to the aspect of the invention, it is preferred that in the recessed portion, the first particles are exposed on the surface.

A sensor according to an aspect of the invention includes the gel for a sensor according to the aspect of the invention and an electrode.

According to this configuration, a sensor capable of easily and stably performing detection of the strength of a stimulus (the concentration or the like of a given component) in a wide range can be provided.

### BRIEF DESCRIPTION OF THE DRAWINGS

The invention will be described with reference to the accompanying drawings, wherein like numbers reference like elements.
FIG. 1 is a schematic longitudinal cross-sectional view for illustrating a sensor according to a first embodiment.
FIG. 2 is a schematic longitudinal cross-sectional view for illustrating a sensor according to a second embodiment.
FIG. 3 is a schematic longitudinal cross-sectional view for illustrating another embodiment of a sensor.

### DESCRIPTION OF EXEMPLARY EMBODIMENTS

Hereinafter, preferred embodiments will be described in detail with reference to the accompanying drawings.

### Sensor and Gel for Sensor

Hereinafter, a sensor (gel sensor) and a gel for a sensor will be described.

### First Embodiment

First, a sensor according to a first embodiment and a gel for a sensor will be described.

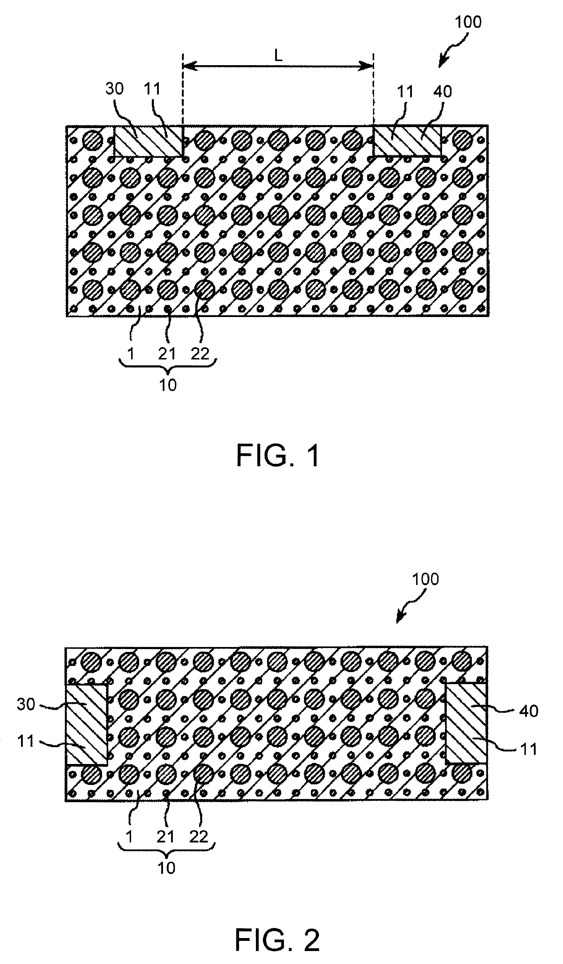

FIG. 1 is a schematic longitudinal cross-sectional view for illustrating a sensor according to a first embodiment. In the following description, a case where the upper side in FIG. 1 is the observer side (viewpoint side) and the lower side in FIG. 1 is a side where a specimen is supplied will be mainly described.

As shown in FIG. 1, a sensor (gel sensor) 100 includes a gel for a sensor 10, a first electrode (electrode) 30, and a second electrode (electrode) 40. Then, the gel for a sensor 10 includes a stimulus-responsive gel 1 which expands or contracts in response to a given stimulus, a plurality of first particles (electrically conductive particles) 21 which are contained in the stimulus-responsive gel 1 and are constituted by a material containing an electrically conductive substance, and a plurality of second particles (particles for structural coloration) 22 which are contained in the stimulus-responsive gel 1 and contribute to structural coloration.

According to such a configuration, a distance between the first particles (electrically conductive particles) 21 contained in the stimulus-responsive gel 1 (in the gel for a sensor 10) can be made different according to the expanded state or the contracted state of the stimulus-responsive gel 1, and as a result, a resistance value between the first electrode 30 and the second electrode 40 can be made different. That is, when the stimulus-responsive gel 1 (gel for a sensor 10) is in an expanded state (or in a state where the degree of expansion is large), the distance between the first particles (electrically conductive particles) 21 is larger than when the stimulus-responsive gel 1 is in a contracted state (or in a state where the degree of expansion is small), and therefore, the resistance value of the gel for a sensor 10 connected to the electrodes (the first electrode 30 and the second electrode 40) is increased. Accordingly, by measuring the resistance value between the first electrode 30 and the second electrode 40, whether the stimulus-responsive gel 1 is in an expanded state or in a contracted state, that is, the strength of a stimulus (the concentration or the like of a given component) can be easily and stably found. In particular, the sensor (gel sensor) 100 is capable of easily and stably performing detection of the strength of a stimulus in a wide range.

Further, by including the second particles (particles for structural coloration) 22, a distance between the second particles (particles for structural coloration) 22 contained in the stimulus-responsive gel 1 (in the gel for a sensor 10) can be changed according to the expanded state or the contracted state of the stimulus-responsive gel 1, and as a result, the structural color caused by Bragg reflection can be made different. That is, when the stimulus-responsive gel 1 (gel for a sensor 10) is in an expanded state (or in a state where the degree of expansion is large), the distance between the second particles (particles for structural coloration) 22 is larger than when the stimulus-responsive gel 1 is in a contracted state (or in a state where the degree of expansion is small), and therefore, the wavelength of the reflected light to be observed becomes longer. Therefore, by observation of the reflected light (including measurement of the wavelength of the reflected light), whether the stimulus-responsive gel 1 is in an expanded state or in a contracted state, that is, the strength of a stimulus (the concentration or the like of a given component) can be easily and stably found. In particular, the sensor (gel sensor) 100 is capable of easily and stably performing detection of the strength of a stimulus in a wide range.

In particular, by combining electrical detection and optical detection, the stimulus detection accuracy can be improved. Further, both simplicity and accuracy of confirmation of the amount of a stimulus can be achieved by visually (intuitively) ascertaining an approximate value (range) of the amount of a given stimulus and thereafter confirming the accurate amount of the stimulus obtained by electrical detection, or the like.

In the case where optical detection is visually performed (in the case where the structural color is visually confirmed), for example, by performing the display of a detection result obtained by electrical detection or the electrical detection itself only when needed (for example, only in a state of reaching a range in which the amount of a stimulus is relatively large), the detection of a given stimulus can be stably performed while reducing the power consumption accompanying the operation of the sensor 100.

By allowing the first particles 21 and the second particles 22 to coexist both in the same stimulus-responsive gel 1, the second particles 22 function as spacers for preventing the first particles 21 from coming excessively close to each other. According to this, even if the first particles 21 are in a state of coming close to each other (a state where the stimulus-responsive gel 1 contracts), a given interval (gap) can be more reliably maintained between the first particles 21, and thus, the detection of a stimulus can be favorably performed in a wider range. In addition, since the first particles 21 can be prevented from coming into contact with each other, undesirable aggregation of the first particles 21 can be prevented, and thus, electrically stable detection of a stimulus can be performed over a long period of time. In particular, the first particles 21 are constituted by a material containing an electrically conductive substance, and therefore, by an electromagnetic action, the first particles 21 move, and a phenomenon that the first particles 21 come into contact with each other is easy to occur, however, by allowing the second particles 22 to function as spacers, the effect as described above can be reliably obtained.

In addition, by allowing the first particles 21 and the second particles 22 to coexist both in the same stimulus-responsive gel 1, the first particles 21 function as spacers for preventing the second particles 22 from coming excessively close to each other. According to this, even if the second particles 22 are in a state of coming close to each other (a state where the stimulus-responsive gel 1 contracts), a given interval (gap) can be more reliably maintained between the second particles 22, and thus, a light with a wavelength which is more easily detected (for example, a light with high visual recognizability) can be reflected. In addition, since the second particles 22 can be prevented from coming into contact with each other, undesirable aggregation of the second particles 22 can be prevented, and thus, optically stable detection of a stimulus can be performed over a long period of time.

In addition, since the first particles 21 and the second particles 22 are dispersed both in the stimulus-responsive gel 1, while preventing the structure and configuration of the sensor 100 and the gel for a sensor 10 from being complicated, the excellent effect as described above is obtained.

### Stimulus-Responsive Gel

The stimulus-responsive gel 1 reversibly expands or contracts in response to a given stimulus.

In this manner, since the stimulus-responsive gel 1 is a material that responds to a given stimulus, the resistance value of the gel for a sensor 10 connected to the electrodes (the first electrode 30 and the second electrode 40) can be changed according to the response (expansion or contraction) of the stimulus-responsive gel 1. As a result, by measuring the resistance value, the presence or absence of the stimulus, the strength (amount or concentration) thereof, etc. can be detected.

The given stimulus to which the stimulus-responsive gel 1 responds varies depending on the constituent material or the like of the stimulus-responsive gel 1, however, examples thereof include various types of substances such as proteins, sugars, uric acid, lactic acid, various types of hormones, various types of ionic substances, and various types of metals, heat, and light.

In the configuration shown in the drawing, the stimulus-responsive gel 1 and the gel for a sensor 10 are in the form of a sheet.

According to this, while reducing the amount of the stimulus-responsive gel 1, the first particles 21, and the second particles 22 to a relatively small amount, the stimulus-responsive gel 1 and the gel for a sensor 10 can be efficiently brought into contact with a specimen, and also the detection of a given stimulus can be easily performed.

The volume of the stimulus-responsive gel 1 included in the sensor 100 is preferably 0.1 mm³ or more and 3600 mm³ or less, more preferably 0.2 mm³ or more and 900 mm³ or less.

According to this, while reducing the size of the sensor 100, the stimulus detection accuracy can be made more excellent. Further, from the viewpoint of resource saving, the above configuration is preferred.

The electrical resistivity of the stimulus-responsive gel 1 alone is preferably 1.0×10⁻² Ω·m or more, more preferably 1.0×10⁻¹ Ω·m or more.

According to this, the difference from the electrical resistivity of the electrically conductive substance constituting the first particles 21 can be made sufficiently large, and the electrical detection to which the first particles 21 contribute can be more favorably performed, and the detection accuracy for the strength (the concentration or the like of a given component) of a stimulus can be made more excellent in a wide range.

The electrical resistivity of the stimulus-responsive gel 1 alone preferably satisfies the conditions as described above when the stimulus-responsive gel 1 is either in an expanded state or in a contracted state, but more preferably satisfies the conditions as described above when the stimulus-responsive gel 1 is both in an expanded state and in a contracted state.

According to this, the above-mentioned effect is more remarkably exhibited.

The refractive index of the stimulus-responsive gel 1 alone is preferably 1.0 or more and 1.8 or less, more preferably 1.1 or more and 1.7 or less.

According to this, the visibility of the structural color caused by Bragg reflection involving the second particles 22 can be made more excellent.

The "refractive index" as used herein refers to an absolute refractive index at 25°C unless otherwise specifically noted.

The refractive index of the stimulus-responsive gel 1 alone preferably satisfies the conditions as described above when the stimulus-responsive gel 1 is either in an expanded state or in a contracted state, but more preferably satisfies the conditions as described above when the stimulus-responsive gel 1 is both in an expanded state and in a contracted state.

According to this, the above-mentioned effect is more remarkably exhibited.

### First Particles

In the stimulus-responsive gel 1, a plurality of first particles (electrically conductive particles) 21 constituted by a material containing an electrically conductive substance are contained.

According to this, a distance between the first particles (electrically conductive particles) 21 changes according to the response (expansion or contraction) of the stimulus-responsive gel 1 to a given stimulus, and the resistance value of the gel for a sensor 10 connected to the electrodes (the first electrode 30 and the second electrode 40) can be changed. As a result, by measuring the resistance value, the presence or absence of the stimulus, the strength (amount or concentration) thereof, etc. can be detected.

Examples of the electrically conductive substance constituting the first particles (electrically conductive particles) 21 include metal materials (for example, Au, Ag, Pt, Cu, an alloy containing at least one member selected from these, and the like), electrically conductive metal oxides (for example, ITO and the like), carbon materials (for example, carbon black and the like), and electrically conductive polymer materials (for example, PEDOT/PSS, a polythiophene-based material, a polyacetylene-based material, and the like).

By including the first particles (electrically conductive particles) 21 constituted by such a material, while making the durability of the sensor 100 excellent, the percentage of change in the resistance value accompanying the expansion or contraction of the stimulus-responsive gel 1 can be further increased, and thus, the stimulus detection accuracy can be made more excellent. In addition, these materials are relatively inexpensive and can be stably obtained, and therefore are advantageous also from the viewpoint of suppression of the production cost of the sensor 100 and stable supply thereof.

In particular, in the case where the first particles (electrically conductive particles) 21 are constituted by a transparent material such as ITO, the structural color involving the second particles 22 is easily distinguished, and the detection of a stimulus can be more favorably performed.

The electrical resistivity of the electrically conductive substance constituting the first particles (electrically conductive particles) 21 is preferably 1.0×10⁻³ Ω·m or less, more preferably 1.0×10⁻⁴ Ω·m or less, further more preferably 5.0×10⁻⁵ Ω·m or less.

According to this, the percentage of change in the resistance value accompanying the expansion or contraction of the stimulus-responsive gel 1 can be further increased, and thus, the stimulus detection accuracy can be made more excellent.

The refractive index of the constituent material of the first particles 21 is preferably 1.0 or more and 1.8 or less, more preferably 1.1 or more and 1.7 or less.

According to this, the visibility of the structural color caused by Bragg reflection involving the second particles 22 can be made more excellent.

The first particle (electrically conductive particle) 21 may have a composition uniform in all regions, or may have a region having a different composition.

For example, the first particle (electrically conductive particle) 21 may be a particle which has a core portion and at least one coating layer provided on the outer surface side of the core portion, or the like.

Further, the first particle (electrically conductive particle) 21 may be a particle subjected to a surface treatment for improving the dispersibility in the stimulus-responsive gel 1.

According to this, an undesirable variation in the composition among the respective regions of the gel for a sensor 10 connected to the electrodes (the first electrode 30 and the second electrode 40) can be suppressed, and thus, the stability of the stimulus detection accuracy can be made more excellent.

Examples of a surface treatment agent which can be used in the surface treatment include agents having a hydrophobic functional group such as an octadecyl group, a hydrophilic functional group such as a hydroxy group, a carboxyl group, or a sulfonic acid group (sulfo group), a salt thereof, or the like in a partial structure. The type of the surface treatment agent can be selected according to, for example, the type or the like of a solvent contained in the stimulus-responsive gel 1.

As described above, in the case where the first particle (electrically conductive particle) 21 has a region having a different composition, the first particle (electrically conductive particle) 21 may have electrical conductivity to such an extent that the effect as described above can be exhibited as a whole, and a portion of the first particle (electrically conductive particle) 21 may be constituted by an insulating material. For example, in the case where at least a portion of the base particle of the first particle (electrically conductive particle) 21 is constituted by a material having electrical conductivity, a portion formed by the surface treatment (a surface-treated portion) may be constituted by an insulating material.

The first particle 21 may have any shape such as a spherical shape, a spindle shape, a needle shape (a spiny shape), a plate shape (a scale shape), or a disk shape such as an erythrocyte shape, but is preferably has a spherical shape.

According to this, the reliability of the electrical resistivity to be detected is further enhanced.

In addition, the first particle 21 may exist in the gel for a sensor 10 as an aggregate formed by aggregating a plurality of particles.

According to this, the electrical conductivity of the gel for a sensor 10 is easily ensured, and thus, the detection of a stimulus by an electrical method can be more favorably performed.

The average particle diameter of the first particles (electrically conductive particles) 21 is not particularly limited, but is preferably 10 nm or more and 1000 µm or less, more preferably 20 nm or more and 500 µm or less.

According to this, in the stimulus-responsive gel 1 (in the gel for a sensor 10), the first particles (electrically conductive particles) 21 can be more uniformly dispersed, and the stimulus detection accuracy can be made more excellent.

In particular, in the case where the average particle diameter of the first particles (electrically conductive particles) 21 is smaller than the average particle diameter of the second particles (particles for structural coloration) 22, the visibility of the structural color is further enhanced.

The average particle diameter of the first particles (electrically conductive particles) 21 in such a case is preferably 10 nm or more and 50 nm or less, more preferably 15 nm or more and 40 nm or less.

According to this, the effect as described above is more remarkably exhibited.

In the case where the average particle diameter of the first particles (electrically conductive particles) 21 is smaller than the average particle diameter of the second particles (particles for structural coloration) 22, when the average particle diameter of the first particles (electrically conductive particles) 21 is represented by D1 (nm), and the average particle diameter of the second particles (particles for structural coloration) 22 is represented by D2 (nm), D1 and D2 preferably satisfy the following relationship: 0.05 ≤ D1/D2 ≤ 0.65, more preferably satisfy the following relationship: 0.1 ≤ D1/D2 ≤ 0.5.

According to this, the effect as described above is more remarkably exhibited.

In addition, in the case where the average particle diameter of the first particles (electrically conductive particles) 21 is larger than the average particle diameter of the second particles (particles for structural coloration) 22, the electrical conductivity of the gel for a sensor 10 is easily ensured, and thus, the detection of a stimulus by an electrical method can be more favorably performed.

The average particle diameter of the first particles (electrically conductive particles) 21 in such a case is preferably 1100 nm or more and 1000 µm or less, more preferably 1500 nm or more and 500 µm or less.

According to this, the effect as described above is more remarkably exhibited.

In the case where the average particle diameter of the first particles (electrically conductive particles) 21 is larger than the average particle diameter of the second particles (particles for structural coloration) 22, when the average particle diameter of the first particles (electrically conductive particles) 21 is represented by D1 (nm), and the average particle diameter of the second particles (particles for structural coloration) 22 is represented by D2 (nm), D1 and D2 preferably satisfy the following relationship: 5 ≤ D1/D2 ≤ 1.5×10⁴, more preferably satisfy the following relationship: 10 ≤ D1/D2 ≤ 7.5×10³.

According to this, the effect as described above is more remarkably exhibited.

The "average particle diameter" as used herein refers to an average particle diameter on the volume basis, and can be obtained by, for example, performing measurement using a particle size distribution analyzer employing a Coulter counter method (model: TA-II, manufactured by Coulter Electronics, Inc.) with an aperture of 50 µm for a dispersion liquid obtained by adding a sample to methanol and dispersing the sample therein for 3 minutes with an ultrasonic disperser.

The gel for a sensor 10 may contain a plurality of types of first particles 21.

The content of the first particles 21 with respect to 100 parts by volume of the stimulus-responsive gel 1 when the stimulus-responsive gel 1 is in an expanded state is preferably 0.1 parts by volume or more and 50 parts by volume or less, more preferably 0.5 parts by volume or more and 40 parts by volume or less.

According to this, the amount of change in the electrical resistivity depending on the state (the expanded state or the contracted state) of the stimulus-responsive gel 1 can be further increased, and thus, the stimulus detection accuracy and detection sensitivity can be made more excellent. Further, when the content of the first particles 21 is a value in the above range, even if the content of the second particles 22 in the gel for a sensor 10 is relatively high, the stimulus-responsive gel 1 can favorably expand or contract. Due to this, a given stimulus can be more favorably detected by an electrical method and an optical method, and the above-mentioned effect is more remarkably exhibited.

In addition, the content of the first particles 21 with respect to 100 parts by volume of the stimulus-responsive gel 1 when the stimulus-responsive gel 1 is in a contracted state is preferably 30 parts by volume or more and 98 parts by volume or less, more preferably 45 parts by volume or more and 90 parts by volume or less.

According to this, the amount of change in the electrical resistivity depending on the state (the expanded state or the contracted state) of the stimulus-responsive gel 1 can be further increased, and thus, the stimulus detection accuracy and detection sensitivity can be made more excellent. Further, when the content of the first particles 21 is a value in the above range, even if the content of the second particles 22 in the gel for a sensor 10 is relatively high, the stimulus-responsive gel 1 can favorably expand or contract. Due to this, a given stimulus can be more favorably detected by an electrical method and an optical method, and the above-mentioned effect is more remarkably exhibited.

### Second Particles

In the stimulus-responsive gel 1, a plurality of second particles 22 are contained.

The second particles 22 contribute to structural coloration caused by Bragg reflection. By including such second particles (particles for structural coloration) 22, when a given stimulus is provided to the stimulus-responsive gel 1, the structural color caused by colloidal crystals or the change in the structural color is easily visually recognized, and therefore, the stimulus can be easily detected by an optical method (including visual observation). Further, since the structural color caused by colloidal crystals or the change in the structural color is easily visually recognized, for example, by the color tone thereof, also the quantitative determination of a given stimulus (the presence or absence of the stimulus, the strength (amount or concentration) thereof) can be easily performed.

Examples of the constituent material of the second particles 22 include inorganic materials such as silica and titanium oxide; and organic materials (polymers) such as polystyrene, polyester, polyimide, polyolefin, poly(methyl (meth)acrylate), polyethylene, polypropylene, polyether sulfone, nylon, polyurethane, polyvinyl chloride, and polyvinylidene chloride, however, the second particles 22 are preferably constituted by silica.

According to this, the shape stability and the like of the second particles 22 are made more excellent, and the durability, reliability, and the like of the gel for a sensor 10 and the sensor 100 can be made more excellent. In addition, the second particles 22 constituted by silica are relatively easily available as those having a sharp particle size distribution (monodispersed fine particles), and therefore are advantageous also from the viewpoint of stable production and supply of the gel for a sensor 10 and the sensor 100. Further, since silica is a material having a high electrical resistivity (for example, 1×10¹¹ Ω·m or more), the second particles 22 can be more effectively prevented from inhibiting the function of the first particles 21 described above.

The electrical resistivity of the constituent material of the second particles 22 is preferably 1.0×10⁶ Ω·m or more, more preferably 1.0×10⁸ Ω·m or more, further more preferably 1.0×10¹⁰ Ω·m or more.

According to this, the difference between the electrical resistivity of the second particles 22 and the electrical resistivity of the first particles 21 can be made sufficiently large, and the second particles 22 can be more effectively prevented from inhibiting the function of the first particles 21 described above.

When the electrical resistivity of the electrically conductive substance constituting the first particles 21 is represented by R1 (Ω·m) and the electrical resistivity of the constituent material of the second particles 22 is represented by R2 (Ω·m), R1 and R2 preferably satisfy the following relationship: 1×10¹² ≤ R2/R1 ≤ 1×10²³, more preferably satisfy the following relationship: 1×10¹³ ≤ R2/R1 ≤ 1×10²².

According to this, the second particles 22 can be more effectively prevented from inhibiting the function of the first particles 21 described above, and the detection of a given stimulus by an electrical method can be more favorably performed.

In addition, as the second particles 22, a high refractive index material having a refractive index of 2.0 or more can be favorably used.

According to this, the change in the structural color can be more remarkably detected.

Examples of the high refractive index material constituting the second particles 22 include titanium oxide, zirconium oxide, and tantalum pentoxide.

The refractive index of the high refractive index material constituting the second particles 22 is preferably 2.1 or more and 2.9 or less, more preferably 2.2 or more and 2.8 or less.

According to this, the effect as described above is more remarkably exhibited.

When the refractive index of the constituent material of the first particles 21 is represented by n1 and the refractive index of the constituent material of the second particles 22 is represented by n2, n1 and n2 preferably satisfy the following relationship: -0.8 ≤ n2-n1 ≤ 1.5, more preferably satisfy the following relationship: -0.7 ≤ n2-n1 ≤ 1.4.

According to this, the structural color can be more clearly caused.

When the refractive index of the constituent material of the first particles 21 is represented by n1 and the refractive index of the constituent material of the second particles 22 is represented by n2, n1 and n2 preferably satisfy the following relationship: 0.1 ≤ |n2-n1|, more preferably satisfy the following relationship: 0.2 ≤ |n2-n1|.

According to this, the structural color can be more clearly caused.

The second particle 22 may have a composition uniform in all regions, or may have a region having a different composition.

For example, the second particle 22 may be a particle which has a core portion and at least one coating layer provided on the outer surface side of the core portion, or the like.

Further, the second particle 22 may be a particle subjected to a surface treatment for improving the dispersibility in the stimulus-responsive gel 1.

According to this, the dispersion stability of the second particles 22 in the stimulus-responsive gel 1 can be made more excellent, and an undesirable variation in the composition among the respective regions in the stimulus-responsive gel 1 can be more effectively suppressed. As a result, the detection of a stimulus by an optical method can be more favorably performed.

Examples of a surface treatment agent which can be used in the surface treatment include agents having a hydrophobic functional group such as an octadecyl group, a hydrophilic functional group such as a hydroxy group, a carboxyl group, or a sulfonic acid group (sulfo group), a salt thereof, or the like in a partial structure. The type of the surface treatment agent can be selected according to, for example, the type or the like of a solvent contained in the stimulus-responsive gel 1.

The second particle 22 may have any shape such as a spherical shape, a spindle shape, a needle shape (a spiny shape), a plate shape (a scale shape), or a disk shape such as an erythrocyte shape, but is preferably has a spherical shape. According to this, the structural color caused by colloidal crystals or the change in the structural color is more favorably visually recognized, and therefore, the detection of a given stimulus can be more easily performed.

The average particle diameter of the second particle 22 is not particularly limited, but is preferably 50 nm or more and 1000 nm or less, more preferably 80 nm or more and 800 nm or less.

According to this, the structural color caused by colloidal crystals or the change in the structural color is more easily visually recognized, and therefore, the detection of a given stimulus can be more easily and also more reliably performed. In addition, since the structural color caused by colloidal crystals or the change in the structural color is more easily visually recognized, for example, by the degree of the change in the color tone, also the quantitative determination of a given stimulus can be more easily and also more accurately performed.

The sensor 100 may contain a plurality of types of second particles 22.

The content of the second particles 22 with respect to 100 parts by volume of the stimulus-responsive gel 1 when the stimulus-responsive gel 1 is in an expanded state is preferably 0.1 parts by volume or more and 50 parts by volume or less, more preferably 0.5 parts by volume or more and 40 parts by volume or less.

According to this, while making the visibility of the structural color more excellent, the degree of the change in the color tone of the structural color depending on the state (the expanded state or the contracted state) of the stimulus-responsive gel 1 can be further increased, and thus, the stimulus detection accuracy and detection sensitivity can be made more excellent. Further, when the content of the second particles 22 is a value in the above range, even if the content of the first particles 21 in the gel for a sensor 10 is relatively high, the stimulus-responsive gel 1 can favorably expand or contract. Due to this, a given stimulus can be more favorably detected by an electrical method and an optical method, and the above-mentioned effect is more remarkably exhibited.

In addition, the content of the second particles 22 with respect to 100 parts by volume of the stimulus-responsive gel 1 when the stimulus-responsive gel 1 is in a contracted state is preferably 30 parts by volume or more and 98 parts by volume or less, more preferably 45 parts by volume or more and 90 parts by volume or less.

According to this, while making the visibility of the structural color more excellent, the degree of the change in the color tone of the structural color depending on the state (the expanded state or the contracted state) of the stimulus-responsive gel 1 can be further increased, and thus, the stimulus detection accuracy and detection sensitivity can be made more excellent. Further, when the content of the second particles 22 is a value in the above range, even if the content of the first particles 21 in the gel for a sensor 10 is relatively high, the stimulus-responsive gel 1 can favorably expand or contract. Due to this, a given stimulus can be more favorably detected by an electrical method and an optical method, and the above-mentioned effect is more remarkably exhibited.

The difference between the electrical resistivity in an expanded state (the maximum electrical resistivity) and the electrical resistivity in a contracted state (the minimum electrical resistivity) of the gel for a sensor 10 is preferably 1.0 × 10⁻⁵ Ω·m or more, more preferably 1.0×10⁻² Ω·m or more.

According to this, the stimulus detection accuracy can be made more excellent.

The thickness of the gel for a sensor 10 when the stimulus-responsive gel 1 is in an expanded state is not particularly limited, but is preferably 5 µm or more and 5000 µm or less, more preferably 7 µm or more and 1000 µm or less.

According to this, while making the durability and reliability of the sensor 100 sufficiently excellent, the thickness and size of the sensor 100 can be reduced. In addition, the flexibility of the sensor 100 can be made more excellent, and for example, even in the case where the sensor 100 is used in close contact with a living body or the like, the adhesiveness of the sensor 100 can be favorably maintained, and the stimulus detection accuracy can be stably made excellent.

The gel for a sensor 10 may have a configuration other than the configuration described above.

For example, the gel for a sensor 10 may be configured to include particles (third particles) other than the first particles and the second particles in the stimulus-responsive gel. In other words, the gel for a sensor 10 may be configured to include insulating particles (third particles) which do not contribute to structural coloration in the stimulus-responsive gel.

In addition, the gel for a sensor 10 may contain a plurality of different types of third particles.

### Electrode

To the gel for a sensor 10 including the stimulus-responsive gel 1 and the first particles 21, the first electrode (electrode) 30 and the second electrode (electrode) 40 are connected.

According to this, the resistance value of the gel for a sensor 10 can be favorably measured, and from this measurement result, the presence or absence of a given stimulus received by the stimulus-responsive gel 1 or the strength thereof can be determined.

The electrodes 30 and 40 are constituted by a material having electrical conductivity.

Examples of the constituent material of the electrodes 30 and 40 include metal materials (for example, Au, Ag, Pt, Cu, an alloy containing at least one member selected from these, and the like), electrically conductive metal oxides (for example, ITO and the like), carbon materials (for example, carbon black and the like), and electrically conductive polymer materials (for example, PEDOT/PSS, a polythiophene-based material, a polyacetylene-based material, and the like).

In particular, an electrically conductive metal oxide such as ITO has transparency and also has excellent durability, and therefore, the state of the gel for a sensor 10 can be favorably observed.

The thickness of the electrodes 30 and 40 is not particularly limited, but is preferably 5 µm or more and 5000 µm or less, more preferably 7 µm or more and 1000 µm or less.

According to this, while making the durability and reliability of the sensor 100 sufficiently excellent, the thickness and size of the sensor 100 can be reduced. In addition, the flexibility of the sensor 100 can be made more excellent, and for example, even in the case where the sensor 100 is used in close contact with a living body or the like, the adhesiveness of the sensor 100 can be favorably maintained, and the stimulus detection accuracy can be stably made excellent.

In this embodiment, a recessed portion 11 is provided in regions coming into contact with the electrodes 30 and 40 of the gel for a sensor 10.

According to this, the positioning of the gel for a sensor 10 and the electrodes 30 and 40 is facilitated, and thus, the stability of detection of a stimulus and the reliability of the sensor 100 can be made more excellent.

In addition, in the configuration shown in the drawing, the electrodes 30 and 40 are provided in contact with the first particles 21 exposed on the surface of the gel for a sensor 10.

According to this, the stability of detection of a stimulus and the reliability of the sensor 100 can be made more excellent.

Further, in this embodiment, the electrodes 30 and 40 are provided on the face of the gel for a sensor 10 on the side opposite to the side where the specimen is supplied.

According to this, for example, even in the case where the specimen is a liquid having electrical conductivity such as sweat, the occurrence of a short circuit due to the specimen existing outside the gel for a sensor 10 can be more effectively prevented, and thus, the reliability of the detection of the stimulus can be made more excellent.

A distance between the electrode 30 and the electrode 40 denoted by L in the drawing (inter-electrode distance) is preferably 0.1 mm or more and 50 mm or less, more preferably 0.2 mm or more and 30 mm or less.

According to this, while suppressing the increase in the size of the sensor 100, the reliability of the detection of a stimulus can be made more excellent.

In addition, the sensor 100 may include an absorbing member (not shown) which absorbs a specimen.

According to this, for example, in the case where an excess amount of a specimen exists outside the gel for a sensor 10, the excess amount of the specimen can be absorbed by the absorbing member, and even in the case where the specimen is a liquid having electrical conductivity such as sweat, the occurrence of a short circuit can be more effectively prevented, and thus, the reliability of the detection of the stimulus can be made more excellent. Further, in the case where a specimen is sequentially supplied (in the case where it is supplied continuously or intermittently), the previously supplied specimen is discharged, and the newly supplied specimen can be supplied to the stimulus-responsive gel 1, and therefore, a change in the amount of the stimulus over time can be found. That is, the detection of the accurate amount of the stimulus can be prevented from being disturbed due to mixing of the previously supplied specimen with the newly supplied specimen. In addition, for example, in the case where the supply of a liquid specimen is stopped or in the case where the supply amount of a specimen is significantly decreased, when the use environment of the sensor 100 is a low humidity environment or the like, the stimulus-responsive gel 1 can be prevented from being dried. As a result, even in an environment in which the stimulus-responsive gel 1 is easily dried, the detection of a given stimulus can be stably performed with high reliability over a relatively long period of time.

The absorbing member may be disposed in any place, but is preferably disposed in a place different from the face of the gel for a sensor 10 on the side where the specimen is supplied.

According to this, while more favorably preventing the supply of the specimen to the stimulus-responsive gel 1 from being inhibited, the effect as described above can be exhibited.

In particular, the absorbing member is preferably disposed on the face of the gel for a sensor 10 on the side opposite to the side where the specimen is supplied.

According to this, the effect as described above is more remarkably exhibited, and in particular, in the case where the specimen is sequentially supplied (in the case where it is supplied continuously or intermittently), the previously supplied specimen is discharged, and the newly supplied specimen can be favorably supplied to the stimulus-responsive gel 1, and therefore, a change in the amount of the stimulus over time can be favorably found.

In addition, the absorbing member is preferably disposed so as not to come into contact with a plurality of electrodes.

According to this, while exhibiting the effect as described above, for example, even in the case where the specimen is a liquid having electrical conductivity such as sweat, the occurrence of a short circuit due to the specimen existing outside the gel for a sensor 10 can be more effectively prevented, and thus, the reliability of the detection of the stimulus can be made more excellent.

Examples of the absorbing member include members constituted by a woven fabric, a non-woven fabric, a cloth-like material such as felt, a porous material, or the like.

As a preferred constituent component of the absorbing member, a cellulose material, a water-absorbing polymer, or the like can be used, however, a cellulose material is particularly preferred. Such a material has moderate hydrophilicity, and therefore, for example, in the case where the specimen contains water (for example, a body fluid such as sweat), the specimen can be favorably absorbed in the material, and also water contained in the absorbed specimen can be favorably evaporated from the material.

### Second Embodiment

Next, a sensor according to a second embodiment will be described.

FIG. 2 is a schematic longitudinal cross-sectional view for illustrating a sensor according to a second embodiment. In the following description, different points from the above-mentioned embodiment will be mainly described, and the description of the same matter will be omitted.

The sensor (gel sensor) 100 of this embodiment has the same configuration as that of the above-mentioned embodiment except that the place where the electrodes 30 and 40 are provided is different. That is, in the above-mentioned embodiment, the electrodes 30 and 40 are provided on the face of the gel for a sensor 10 on the side opposite to the side where the specimen is supplied, however, in the sensor (gel sensor) 100 of this embodiment, the electrodes 30 and 40 are provided in side face portion of the gel for a sensor 10. In this manner, the place where the electrodes are provided may be any place. In particular, as in this embodiment, by providing the electrodes 30 and 40 in a side face portion of the gel for a sensor 10 in the form of a sheet including the stimulus-responsive gel 1, the first particles 21, and the second particles 22, the thickness of the sensor 100 can be reduced.

In the configuration shown in FIG. 2, the electrodes 30 and 40 are provided only in a portion in the thickness direction of the gel for a sensor 10, but may be provided throughout the thickness direction of the gel for a sensor 10.

### Constituent Material of Stimulus-Responsive Gel

Next, the constituent material of the stimulus-responsive gel constituting the sensor will be described.

The stimulus-responsive gel may be constituted by any material as long as it responds to a given stimulus, but is generally constituted by a material containing a polymer material having a crosslinked structure and a solvent.

### Polymer Material

The polymer material constituting the stimulus-responsive gel is an important component for detecting a specific stimulus, and the structure thereof varies depending on the type of the stimulus to be detected.

The polymer material constituting the stimulus-responsive gel is not particularly limited, and can be selected according to the stimulus to be detected.

Hereinafter, specific examples of the polymer material constituting the stimulus-responsive gel will be described.

As the polymer material constituting the stimulus-responsive gel, for example, a polymer material obtained by reacting a monomer, a polymerization initiator, a crosslinking agent, etc. can be used.

Examples of the monomer include acrylamide, N-methylacrylamide, N-isopropylacrylamide, N,N-dimethylacrylamide, N,N-dimethylaminopropylacrylamide, various quaternary salts of N,N-dimethylaminopropylacrylamide, acryloylmorpholine, various quaternary salts of N,N-dimethylaminoethylacrylate, acrylic acid, various alkyl acrylates, methacrylic acid, various alkyl methacrylates, 2-hydroxyethylmethacrylate, glycerol monomethacrylate, N-vinylpyrrolidone, acrylonitrile, styrene, polyethylene glycol diacrylate, 1,6-hexanediol diacrylate, neopentyl glycol diacrylate, tripropylene glycol diacrylate, polypropylene glycol diacrylate, 2,2-bis[4-(acryloxydiethoxy)phenyl]propane, 2,2-bis[4-(acryloxypolyethoxy)phenyl]propane, 2-hydroxy-1-acryloxy-3-methacryloxypropane, 2,2-bis[4-(acryloxypolypropoxy)phenyl]propane, ethylene glycol dimethacrylate, diethylene glycol dimethacrylate, triethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2-hydroxy-1,3-dimethacryloxypropane, 2,2-bis[4-(methacryloxyethoxy)phenyl]propane, 2,2-bis[4-(methacryloxyethoxydiethoxy)phenyl]propane, 2,2-bis[4-(methacryloxyethoxypolyethoxy)phenyl]propane, trimethylolpropane trimethacrylate, tetramethylolmethane trimethacrylate, trimethylolpropane triacrylate, tetramethylolmethane triacrylate, tetramethylolmethane tetraacrylate, dipentaerythritol hexaacrylate, N,N'-methylenebisacrylamide, N,N'-methylenebismethacrylamide, diethylene glycol diallyl ether, and divinylbenzene.

Further, examples of a functional group which can interact with a sugar include a boronic acid group (particularly, a phenylboronic acid group), and therefore, a monomer having a boronic acid group may be used. Examples of such a boronic acid group-containing monomer include acryloylaminobenzeneboronic acid, methacryloylaminobenzeneboronic acid, and 4-vinylbenzeneboronic acid.

In the case where an ionic substance (particularly, an ionic substance containing a calcium ion) is detected as a stimulus (specific component), a crown ether group-containing monomer (particularly, a benzocrown ether group-containing monomer) such as 4-acrylamidobenzo-18-crown 6-ether, acryloyl aminobenzocrown ether, methacryloyl aminobenzocrown ether, or 4-vinylbenzocrown ether can be preferably used as the monomer.

In the case where an ionic substance such as sodium chloride is detected as a stimulus (specific component), 3-acrylamidophenylboronic acid, vinylphenylboronic acid, acryloyloxyphenylboronic acid, N-isopropylacrylamide (NIPAAm), ethylenebisacrylamide, N-hydroxyethylacrylamide, or the like can be preferably used as the monomer. In particular, in the case where an ionic substance such as sodium chloride is detected as a stimulus (specific component), it is preferred to use at least one monomer selected from the group consisting of 3-acrylamidophenylboronic acid, vinylphenylboronic acid, and acryloyloxyphenylboronic acid, and at least one monomer selected from the group consisting of N-isopropylacrylamide (NIPAAm), ethylenebisacrylamide, and N-hydroxyethylacrylamide in combination as the monomer.

In the case where lactic acid is detected as a stimulus (specific component), 3-acrylamidophenylboronic acid, vinylphenylboronic acid, acryloyloxyphenylboronic acid, N-isopropylacrylamide (NIPAAm), ethylenebisacrylamide, N-hydroxyethylacrylamide, or the like can be preferably used as the monomer. In particular, in the case where lactic acid is detected as a stimulus (specific component), it is preferred to use at least one monomer selected from the group consisting of 3-acrylamidophenylboronic acid, vinylphenylboronic acid, and acryloyloxyphenylboronic acid, and at least one monomer selected from the group consisting of N-isopropylacrylamide (NIPAAm), ethylenebisacrylamide, and N-hydroxyethylacrylamide in combination as the monomer.

The polymerization initiator can be appropriately selected according to, for example, the polymerization method thereof. Specific examples thereof include compounds which generate radicals by ultraviolet light including hydrogen peroxide, persulfates such as potassium persulfate, sodium persulfate, and ammonium persulfate, azo-based initiators such as 2,2'-azobis(2-amidinopropane) dihydrochloride, 2,2'-azobis(N,N'-dimethyleneisobutylamidine) dihydrochloride, 2,2'-azobis{2-methyl-N-[1,1-bis(hydroxymethyl)-2-hydroxyet hyl]propionamide}, 2,2'-azobis[2-(2-imidazolin-2-yl)propane] dihydrochloride, 4,4'-azobis(4-cyanovaleric acid), 2,2'-azobisisobutyronitrile, 2,2'-azobis(2,4'-dimethylvaleronitrile), benzophenone, 2,2-dimethoxy-1,2-diphenylethan-1-one, 1-hydroxycyclohexyl phenyl ketone, 2-hydroxy-2-methyl-1-phenylpropan-1-one, 2,4,6-trimethylbenzoyl diphenylphosphine oxide, 1-[4-(2-hydroxyethoxy)-phenyl]-2-hydroxy-2-methyl-1-propan -1-one, and the like, and compounds which generate radicals by light with a wavelength of 360 nm or more such as substances obtained by mixing a thiopyrylium salt-based, merocyanine-based, quinoline-based, orstyrylquinoline-based dye with 2,4-diethyl thioxanthone, isopropyl thioxanthone, 1-chloro-4-propoxythioxanthone, 2-(3-dimethylamino-2-hydroxypropoxy)-3,4-dimethyl-9H-thiox anthon-9-one methochloride, 2-methyl-1[4-(methylthio)phenyl]-2-morpholinopropane-1,2-b enzyl-2-dimethylamino-1-(4-morpholinophenyl)-butanone-1, bis(cyclopentadienyl)-bis(2,6-difluoro-3-(pyl-1-yl) titanium, or a peroxy ester such as 1,3-di(t-butylperoxycarbonyl)benzene or 3,3',4,4'-tetra-(t-butylperoxycarbonyl)benzophenone. Hydrogen peroxide or a persulfate can also be used as a redox-based initiator in combination with, for example, a reducing substance such as a sulfite or L-ascorbic acid, an amine salt, or the like.

As the crosslinking agent, a compound having two or more polymerizable functional groups can be used, and specific examples thereof include ethylene glycol, propylene glycol, trimethylolpropane, glycerin, polyoxyethylene glycol, polyoxypropylene glycol, polyglycerin, N,N'-methylenebisacrylamide, N,N-methylene-bis-N-vinylacetamide, N,N-butylene-bis-N-vinylacetamide, tolylene diisocyanate, hexamethylene diisocyanate, allylated starch, allylated cellulose, diallyl phthalate, tetraallyloxyethane, pentaerythritol triallyl ether, trimethylolpropane triallyl ether, diethylene glycol diallyl ether, and triallyl trimellitate.

The stimulus-responsive gel may contain a plurality of different types of polymer materials.

For example, the stimulus-responsive gel may contain as the polymer material, a first polymer having an OH group (a hydroxy group which bonds to a carbon atom) and a second polymer having a phenylboronic acid structure.

According to this, the stimulus-responsive gel can be in a first state where the OH group possessed by the first polymer and the phenylboronic acid structure possessed by the second polymer are bonded to each other, and in a second state where the bond between the OH group possessed by the first polymer and the phenylboronic acid structure possessed by the second polymer is dissociated.

The change between these states occurs by a change in the surrounding environment (a change in the presence or absence of a given stimulus or a change in the strength or the like of a stimulus). Then, the ratio of molecules which are in the first state to molecules which are in the second state among the many molecules (the first polymer molecules and the second polymer molecules) contained in the stimulus-responsive gel changes with inclination according to the strength of the stimulus.

Due to this, the strength of the stimulus can be quantitatively detected.

It is preferred that the stimulus-responsive gel is transformed to the second state by reacting the phenylboronic acid structure possessed by the second polymer with lactic acid.

The stimulus-responsive gel containing the first polymer and the second polymer as described above can detect and quantitatively determine lactic acid among various stimuli with particularly high sensitivity in a particularly wide concentration range. In the past, the detection and quantitative determination of lactic acid were mostly performed using an enzyme, and there was no gel material capable of being favorably applied to the detection and quantitative determination of lactic acid. In view of this, by using the stimulus-responsive gel for the detection and quantitative determination of lactic acid, the effect of the invention is more remarkably exhibited.

The reason why the stimulus-responsive gel (the stimulus-responsive gel containing the first polymer and the second polymer) as described above shows high sensitivity to lactic acid is considered to be as follows. That is, in the case where the sensor is used for detecting lactic acid, when the concentration of lactic acid is low, the ratio of molecules in the first state where the OH group possessed by the first polymer and the phenylboronic acid structure possessed by the second polymer are bonded to each other is increased. On the other hand, when the concentration of lactic acid is increased, the bond between the OH group possessed by the first polymer and the phenylboronic acid structure possessed by the second polymer is replaced by a bond between lactic acid and the phenylboronic acid structure with extremely high reactivity. This is considered to be because lactic acid is a compound which has an α-hydroxycarboxylic acid structure and has particularly high reactivity with a phenylboronic acid structure, and also the size of the molecule is small, and therefore, in the stimulus-responsive gel, lactic acid can easily come close to the phenylboronic acid structure possessed by the second polymer. In addition, by bonding lactic acid to the phenylboronic acid structure constituting the second polymer, the hydrophilicity of the second polymer largely changes, and due to this, the expansion coefficient of the stimulus-responsive gel can be further increased. As a result, the stimulus (lactic acid) detection sensitivity and detection accuracy can be made more excellent.

Hereinafter, a case where the stimulus-responsive gel contains the first polymer having an OH group (a hydroxy group which bonds to a carbon atom) and the second polymer having a phenylboronic acid structure, in particular, a case where the stimulus-responsive gel is transformed to the second state by reacting the phenylboronic acid structure possessed by the second polymer with lactic acid and is used for the detection and quantitative determination of lactic acid will be mainly described.

### First Polymer

The first polymer has an OH group (hydroxy group).

The OH group may be an OH group introduced after polymerizing a polymerizable compound (a monomer or the like) as a constituent component of the polymer, but is preferably an OH group possessed by a polymerizable compound as a constituent component of the polymer.

According to this, the adjustment of the percentage or the like of the OH group possessed by the first polymer can be easily and reliably performed.

Examples of the monomer having an OH group which constitutes the first polymer include N-hydroxyethyl acrylamide, N,N'-(1,2-dihydroxyethylene)bisacrylamide, 2-hydroxyethyl methacrylate, glycerol monomethacrylate, 2-hydroxy-1-acryloxy-3-methacryloxypropane, and 2-hydroxy-1,3-dimethacryloxypropane, and it is possible to use one member or two or more members in combination selected from these, however, the first polymer preferably contains N-hydroxyethyl acrylamide as a constituent component.

According to this, the transformation between the first state and the second state according to the change in the environment (particularly the change in the concentration of lactic acid) in which the stimulus-responsive gel is placed is more favorably performed, and the detection and quantitative determination of the strength of a stimulus (particularly, the concentration of lactic acid) can be more stably performed in a wider range. In addition, the ability to retain the solvent of the stimulus-responsive gel can be made particularly excellent, and thus, a favorable gel state can be stably maintained over a long period of time.

The first polymer may contain a monomer having no OH group as a constituent component thereof. According to this, the percentage or the like of the OH group possessed by the first polymer can be adjusted to a favorable level.

Examples of the monomer having no OH group which constitutes the first polymer include acrylamide, N-methylacrylamide, N-isopropylacrylamide, N,N-dimethylacrylamide, N,N-dimethylaminopropyl acrylamide, various quaternary salts of N,N-dimethylaminopropyl acrylamide, acryloylmorpholine, various quaternary salts of N,N-dimethylaminoethyl acrylate, acrylic acid, various alkyl acrylates, methacrylic acid, various alkyl methacrylates, N-vinylpyrrolidone, acrylonitrile, styrene, polyethyleneglycol diacrylate, 1,6-hexanediol diacrylate, neopentyl glycol diacrylate, tripropylene glycol diacrylate, polypropylene glycol diacrylate, 2,2-bis[4-(acryloxydiethoxy)phenyl]propane, 2,2-bis[4-(acryloxypolyethoxy)phenyl]propane, 2,2-bis[4-(acryloxypolypropoxy)phenyl]propane, ethylene glycol dimethacrylate, diethylene glycol dimethacrylate, triethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2,2-bis[4-(methacryloxyethoxy)phenyl]propane, 2,2-bis[4-(methacryloxyethoxydiethoxy)phenyl]propane, 2,2-bis[4-(methacryloxyethoxypolyethoxy)phenyl]propane, trimethylolpropane trimethacrylate, tetramethylolmethane trimethacrylate, trimethylolpropane triacrylate, tetramethylolmethane triacrylate, tetramethylolmethane tetraacrylate, dipentaerythritol hexaacrylate, N,N'-methylene bisacrylamide, N,N'-methylene bismethacrylamide, diethylene glycol diallyl ether, divinylbenzene, ethylenebisacrylamide, N-[3-(dimethylamino)propyl]methacrylamide, diacetone acrylamide, N-t-butylacrylamide, N,N-diethylacrylamide, N-isopropylmethacrylamide, N-(butoxymethyl)acrylamide, N-(isobutoxymethyl)acrylamide, N-phenylacrylamide, 2-acrylamide-2-methylpropanesulfonic acid, 4-acrylamidobenzo-18-crown 6-ether, acryloylaminobenzocrown ether, methacryloylaminobenzocrown ether, and 4-vinylbenzocrown ether, and it is possible to use one member or two or more members in combination selected from these.

The content of the monomer having no OH group in the first polymer is preferably 0.1 mol% or more and 40 mol% or less, more preferably 0.2 mol% or more and 30 mol% or less, further more preferably 0.3 mol% or more and 20 mol% or less.

The first polymer may contain a crosslinking agent component as a constituent component.

According to this, the first polymer has a crosslinked structure, and thus has a three-dimensional network structure. As a result, the ability to retain the solvent of the stimulus-responsive gel can be made particularly excellent, and thus, a favorable gel state can be stably maintained over a long period of time. That is, the stimulus-responsive gel has excellent durability.

As the crosslinking agent component, a compound having two or more polymerizable functional groups can be used, and specific examples thereof include ethylene glycol, propylene glycol, trimethylol propane, glycerin, polyoxyethylene glycol, polyoxypropylene glycol, polyglycerin, N,N'-methylene bisacrylamide, N,N-methylene-bis-N-vinylacetamide, N,N-butylene-bis-N-vinylacetamide, tolylene diisocyanate, hexamethylene diisocyanate, allylated starch, allylated cellulose, diallyl phthalate, tetraallyloxyethane, pentaerythritol triallyl ether, trimethylol propane triallyl ether, diethylene glycol diallyl ether, and triallyl trimellitate, and it is possible to use one member or two or more members in combination selected from these.

The content of the crosslinking agent component in the first polymer is preferably 0.5 mol% or more and 7.0 mol% or less, more preferably 0.8 mol% or more and 6.0 mol% or less, further more preferably 1.1 mol% or more and 5.0 mol% or less.

According to this, the degree of crosslinking of the first polymer can be made to fall within a more favorable range, and while more remarkably exhibiting the effect as described above, the flexibility of the first polymer can be made more suitable.

The content of the monomer having an OH group in the first polymer is preferably 54 mol% or more and 99 mol% or less, more preferably 65 mol% or more and 98.5 mol% or less, further more preferably 76 mol% or more and 98 mol% or less.

According to this, while more remarkably exhibiting the effect of the first polymer because of having an OH group as described above, the effect of the components (the crosslinking agent, the monomer having no OH group, etc.) other than the monomer having an OH group can be sufficiently exhibited.

The hydroxyl value of the first polymer is preferably 15 mgKOH/g or more and 620 mgKOH/g or less, more preferably 34 mgKOH/g or more and 78 mgKOH/g or less.

According to this, while more remarkably exhibiting the effect of the first polymer because of having an OH group as described above, the durability of the stimulus-responsive gel can be made particularly excellent.

On the other hand, when the hydroxyl value of the first polymer is less than the above lower limit, the effect of the first polymer because of having an OH group as described above may not be sufficiently obtained depending on the percentage or the like of the phenylboronic acid structure possessed by the second polymer.

Further, when the hydroxyl value of the first polymer exceeds the above upper limit, the durability of the stimulus-responsive gel is decreased.

The first polymer does not have a phenylboronic acid structure.

The content X₁ of the first polymer in the stimulus-responsive gel is preferably 0.05 mass% or more and 98 mass% or less, more preferably 0.1 mass% or more and 70 mass % or less.

According to this, the sensitivity and quantitativeness with respect to lactic acid can be made particularly excellent, and also the ability to retain the solvent of the stimulus-responsive gel can be made particularly excellent, and thus, a favorable gel state can be stably maintained over a long period of time.

In addition, the content of the first polymer in the polymer material is preferably 1.0 mass% or more and 99 mass% or less, more preferably 1.5 mass% or more and 98 mass% or less.

According to this, particularly excellent sensitivity and quantitativeness with respect to lactic acid are obtained, and also the ability to retain the solvent of the stimulus-responsive gel can be made particularly excellent, and thus, a favorable gel state can be stably maintained over a long period of time.

### Second Polymer

The second polymer has a phenylboronic acid structure.

By including such a second polymer along with the above-mentioned first polymer, the detection of the strength of a stimulus (the concentration or the like of a given component) in a wide range can be easily and stably performed. In particular, in the case where the sensor is used for performing the detection and quantitative determination of lactic acid, the sensitivity in a low concentration range (for example, in a range of 0.4 mass% or less) can be made excellent.

The phenylboronic acid structure may be a phenylboronic acid structure introduced after polymerizing a polymerizable compound (a monomer or the like) as a constituent component of the polymer, but is preferably a phenylboronic acid structure possessed by a polymerizable compound as a constituent component of the polymer.

According to this, the adjustment of the percentage or the like of the phenylboronic acid structure possessed by the second polymer can be easily and reliably performed.

Examples of the monomer having a phenylboronic acid structure which constitutes the second polymer include 3-acrylamidophenylboronic acid, vinylphenylboronic acid, acryloyloxyphenylboronic acid, acryloylaminobenzeneboronic acid, methacryloylaminobenzeneboronic acid, and 4-vinylbenzeneboronic acid, and it is possible to use one member or two or more members in combination selected from these, however, the second polymer preferably contains 3-acrylamidophenylboronic acid as a constituent component.

According to this, the transformation between the first state and the second state according to the change in the environment (particularly the change in the concentration of lactic acid) in which the stimulus-responsive gel is placed is more favorably performed, and the detection and quantitative determination of the strength of a stimulus (particularly, the concentration of lactic acid) can be more stably performed in a wider range. In addition, the ability to retain the solvent of the stimulus-responsive gel can be made particularly excellent, and thus, a favorable gel state can be stably maintained over a long period of time.

The second polymer may contain a monomer having no phenylboronic acid structure as a constituent component thereof. According to this, the percentage or the like of the phenylboronic acid structure possessed by the second polymer can be adjusted to a favorable level.

Examples of the monomer having no phenylboronic acid structure which constitutes the second polymer include acrylamide, N-methylacrylamide, N-isopropylacrylamide, N,N-dimethylacrylamide, N,N-dimethylaminopropyl acrylamide, various quaternary salts of N,N-dimethylaminopropyl acrylamide, acryloylmorpholine, various quaternary salts of N,N-dimethylaminoethyl acrylate, acrylic acid, various alkyl acrylates, methacrylic acid, various alkyl methacrylates, N-vinylpyrrolidone, acrylonitrile, styrene, polyethyleneglycol diacrylate, 1,6-hexanediol diacrylate, neopentyl glycol diacrylate, tripropylene glycol diacrylate, polypropylene glycol diacrylate, 2,2-bis[4-(acryloxydiethoxy)phenyl]propane, 2,2-bis[4-(acryloxypolyethoxy)phenyl]propane, 2,2-bis[4-(acryloxypolypropoxy)phenyl]propane, ethylene glycol dimethacrylate, diethylene glycol dimethacrylate, triethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2,2-bis[4-(methacryloxyethoxy)phenyl]propane, 2,2-bis[4-(methacryloxyethoxydiethoxy)phenyl]propane, 2,2-bis[4-(methacryloxyethoxypolyethoxy)phenyl]propane, trimethylolpropane trimethacrylate, tetramethylolmethane trimethacrylate, trimethylolpropane triacrylate, tetramethylolmethane triacrylate, tetramethylolmethane tetraacrylate, dipentaerythritol hexaacrylate, N,N'-methylene bisacrylamide, N,N'-methylene bismethacrylamide, diethylene glycol diallyl ether, divinylbenzene, ethylenebisacrylamide, N-[3-(dimethylamino)propyl]methacrylamide, diacetone acrylamide, N-t-butylacrylamide, N,N-diethylacrylamide, N-isopropylmethacrylamide, N-(butoxymethyl)acrylamide, N-(isobutoxymethyl)acrylamide, N-phenylacrylamide, N-hydroxyethylacrylamide, 2-hydroxyethylmethacrylate, glycerol monomethacrylate, 2-hydroxy-1-acryloxy-3-methacryloxypropane, 2-hydroxy-1,3-dimethacryloxypropane, 4-acrylamidobenzo-18-crown 6-ether, acryloylaminobenzocrown ether, methacryloylaminobenzocrown ether, and 4-vinylbenzocrown ether, and it is possible to use one member or two or more members in combination selected from these, however, the second polymer preferably contains N-hydroxyethylacrylamide as a constituent component.

According to this, the affinity between the first polymer and the second polymer can be made more favorable, and a problem of undesirable phase separation or the like in the stimulus-responsive gel can be more reliably prevented over a longer period of time, and the durability and reliability of the stimulus-responsive gel can be made particularly excellent. In addition, the ability to retain the solvent of the stimulus-responsive gel can be made particularly excellent.

The content of the monomer having no phenylboronic acid structure in the second polymer is preferably 1 mol% or more and 96 mol% or less, more preferably 5 mol% or more and 95 mol% or less, further more preferably 20 mol% or more and 93 mol% or less.

The second polymer may contain a crosslinking agent component as a constituent component.

According to this, the second polymer has a crosslinked structure, and thus has a three-dimensional network structure. As a result, the ability to retain the solvent of the stimulus-responsive gel can be made particularly excellent, and thus, a favorable gel state can be stably maintained over a long period of time. That is, the stimulus-responsive gel has excellent durability.

As the crosslinking agent component, a compound having two or more polymerizable functional groups can be used, and specific examples thereof include ethylene glycol, propylene glycol, trimethylol propane, glycerin, polyoxyethylene glycol, polyoxypropylene glycol, polyglycerin, N,N'-methylene bisacrylamide, N,N-methylene-bis-N-vinylacetamide, N,N-butylene-bis-N-vinylacetamide, tolylene diisocyanate, hexamethylene diisocyanate, allylated starch, allylated cellulose, diallyl phthalate, tetraallyloxyethane, pentaerythritol triallyl ether, trimethylol propane triallyl ether, diethylene glycol diallyl ether, and triallyl trimellitate, and it is possible to use one member or two or more members in combination selected from these.

The content of the crosslinking agent component in the second polymer is preferably 0.5 mol% or more and 10.0 mol% or less, more preferably 0.8 mol% or more and 8.0 mol% or less, further more preferably 1.1 mol% or more and 6.0 mol% or less.

According to this, the degree of crosslinking of the second polymer can be made to fall within a more favorable range, and while more remarkably exhibiting the effect as described above, the flexibility of the second polymer can be made more suitable.

The content of the monomer having a phenylboronic acid structure in the second polymer is preferably 3.0 mol% or more and 98 mol% or less, more preferably 3.5 mol% or more and 70 mol% or less, further more preferably 3.8 mol% or more and 70 mol% or less.

According to this, the stimulus-responsive gel has particularly excellent flexibility, and therefore has particularly excellent sensitivity and quantitativeness with respect to a given stimulus, and the detection and quantitative determination of the strength of a stimulus (particularly, the concentration of lactic acid) can be more stably performed in a wider range.

On the other hand, when the content of the monomer having a phenylboronic acid structure in the second polymer is less than the above lower limit, it may be difficult to make the range (for example, the concentration of lactic acid) in which the detection and quantitative determination of the strength of a stimulus (for example, the concentration of lactic acid) can be favorably performed sufficiently wide depending on the percentage or the like of the OH group possessed by the first polymer.

In addition, when the content of the monomer having a phenylboronic acid structure in the second polymer exceeds the above upper limit, the stimulus-responsive gel is hard to deform, and therefore, the sensitivity and quantitativeness with respect to a given stimulus are deteriorated.

This is considered to be because the percentage of the phenylboronic acid structure is increased, so that the π-π electron interaction (π-π stacking) between a plurality of benzene rings strongly appears, and therefore a space into which the solvent or the like enters is reduced.

The content X₂ of the second polymer in the stimulus-responsive gel is preferably 0.01 mass% or more and 70 mass% or less, more preferably 0.05 mass% or more and 65 mass% or less.

According to this, the stimulus-responsive gel has particularly excellent flexibility, and therefore has particularly excellent sensitivity and quantitativeness with respect to a given stimulus, and the detection and quantitative determination of the strength of a stimulus (particularly, the concentration of lactic acid) can be more stably performed in a wider range.

On the other hand, when the content X₂ of the second polymer in the stimulus-responsive gel is less than the above lower limit, it may be difficult to make the range (for example, the concentration of lactic acid) in which the detection and quantitative determination of the strength of a stimulus (for example, the concentration of lactic acid) can be favorably performed sufficiently wide depending on the percentage or the like of the OH group possessed by the first polymer.

In addition, when the content X₂ of the second polymer in the stimulus-responsive gel exceeds the above upper limit, the stimulus-responsive gel is hard to deform, and therefore, the sensitivity and quantitativeness with respect to a given stimulus are deteriorated.

Further, the content of the second polymer in the polymer material is preferably 1.0 mass% or more and 70 mass% or less, more preferably 2.0 mass% or more and 65 mass% or less.

According to this, the stimulus-responsive gel has particularly excellent flexibility, and therefore has particularly excellent sensitivity and quantitativeness with respect to a given stimulus, and the detection and quantitative determination of the strength of a stimulus (particularly, the concentration of lactic acid) can be more stably performed in a wider range.

On the other hand, when the content of the second polymer in the polymer material is less than the above lower limit, it may be difficult to make the range (for example, the concentration of lactic acid) in which the detection and quantitative determination of the strength of a stimulus (for example, the concentration of lactic acid) can be favorably performed sufficiently wide depending on the percentage or the like of the OH group possessed by the first polymer.

In addition, when the content of the second polymer in the polymer material exceeds the above upper limit, the stimulus-responsive gel is hard to deform, and therefore, the sensitivity and quantitativeness with respect to a given stimulus are deteriorated.

When the content of the first polymer in the stimulus-responsive gel is represented by X₁ (mass%) and the content of the second polymer in the stimulus-responsive gel is represented by X₂ (mass%), X₁ and X₂ preferably satisfy the following relationship: 0.2 ≤ X₂/X₁ ≤ 8, more preferably satisfy the following relationship: 1.3 ≤ X₂/X₁ ≤ 1.9.

According to this, the stimulus-responsive gel has particularly excellent flexibility, and therefore has particularly excellent sensitivity and quantitativeness with respect to a given stimulus, and the detection and quantitative determination of the strength of a stimulus (particularly, the concentration of lactic acid) can be more stably performed in a wider range.

On the other hand, when the value of X₂/X₁ is less than the above lower limit, it may be difficult to make the range (for example, the concentration of lactic acid) in which the detection and quantitative determination of the strength of a stimulus (for example, the concentration of lactic acid) can be favorably performed sufficiently wide.

In addition, when the value of X₂/X₁ exceeds the above upper limit, the stimulus-responsive gel is hard to deform, and therefore, the sensitivity and quantitativeness with respect to a given stimulus are deteriorated.

The content of the polymer material in the stimulus-responsive gel is preferably 0.7 mass% or more and 36.0 mass% or less, more preferably 2.4 mass% or more and 27.0 mass% or less.

### Solvent

By including the solvent in the stimulus-responsive gel, the above-mentioned polymer material can be favorably gelled.

As the solvent, any of various types of organic solvents and inorganic solvents can be used. Specific examples thereof include water; various types of alcohols such as methanol and ethanol; ketones such as acetone; ethers such as tetrahydrofuran and diethyl ether; amides such as dimethylformamide; chain aliphatic hydrocarbons such as n-pentane, n-hexane, n-heptane, and n-octane; alicyclic hydrocarbons such as cyclohexane and methylcyclohexane; and aromatic hydrocarbons such as benzene, toluene, and xylene, however, in particular, a solvent containing water is preferred.

The stimulus-responsive gel may contain a plurality of different types of components as the solvent.

The content of the solvent in the stimulus-responsive gel is preferably 30 mass% or more and 95 mass% or less, more preferably 50 mass% or more and 90 mass% or less.

### Another Component

The stimulus-responsive gel may contain a component other than the above-mentioned components (another component).

### Application of Sensor

The sensor can easily detect a given stimulus (for example, a specific component), and therefore can be used as, for example, a sensor for determining whether or not a specific substance is contained in a test subject (a specimen) or measuring the concentration of a specific substance contained a test subject.

Further, the sensor can easily detect the amount of a specific component incorporated in the stimulus-responsive gel, and therefore can also be favorably used as a separation and extraction unit that separates and extracts a specific substance contained in a test subject. That is, at a stage where the amount of a specific component incorporated in the stimulus-responsive gel is saturated or almost saturated, the contact thereof with the test subject is stopped, and according to need, it can be replaced by another sensor. According to this, the specific component can be collected from the test subject without any waste.

More specific application of the sensor include, for example, sensors for biological substances (for example, various types of cells such as cancer cells and blood cells, proteins such as antibodies (including glycoproteins and the like), etc.), sensors for components (for example, lactic acid, uric acid, sugars, etc.) contained in body fluids or substances secreted outside the body (for example, blood, saliva, sweat, urine, etc.), separation and extraction units for biological substances (particularly, trace biological substances and the like such as hormones), separation and extraction units for metals (particularly, rare metals, noble metals, etc.), sensors for antigens (allergic substances) such as pollens, separation and extraction units for poisons, toxic substances, environmental pollutants, etc., sensors for viruses, bacteria, etc., sensors for components contained in soils, sensors for components contained in waste fluids (including drained water), sensors for components contained in foods, sensors for components contained in water (for example, salts and the like contained in brackish waters, rivers, paddies, etc.), cell culture monitors, and the like.

Further, the sensor is preferably a sensor to be used in close contact with the skin of a living body.

The skin of a living body generally has a complicated rugged shape, however, as described above, the sensor has excellent shape followability, and therefore can be favorably brought into close contact with the skin of a living body. Further, in the case where the sensor is used in close contact with the skin of a living body (for example, a case where a component contained in sweat when an exercise is performed is detected as a stimulus (specific component), or the like), it is assumed that a large external force such as vibration or impact is applied to the sensor. However, even in such a case that a relatively large external force is applied to the sensor, a specific component (given stimulus) can be accurately detected. Therefore, the effect is more remarkably exhibited in the case where the sensor is used in close contact with the skin of a living body.

Further, the sensor can be also favorably applied to reduction in size and weight. Accordingly, the sensor is suitable for use in the method as described above.

### Method of Using Sensor

Hereinafter, one example of a method of using the sensor will be specifically described.

First, the sensor 100 when the stimulus-responsive gel 1 is in a state where it does not receive a given stimulus (for example, in the case where the given stimulus is a specific component, a state where the stimulus-responsive gel 1 does not contain the specific component) is prepared.

Subsequently, the electrodes 30 and 40 are electrically connected to a unit that measures an electrical resistance, and the resistance value in a state where the stimulus-responsive gel 1 does not receive a given stimulus is measured (initialization). At this time, in the case where the given stimulus is a specific substance, for example, calibration may be performed using standard solutions containing the specific substance at given concentrations. By doing this, the stimulus detection accuracy can be further enhanced.

Thereafter, the detection of the stimulus is performed in a state where the specimen and the sensor 100 can come into contact with each other.

By performing the measurement in such a manner, the detection of the strength of a stimulus (the concentration or the like of a given component) can be easily and stably performed in a wide range.

The sensor 100 is preferably a sensor which constitutes a part of a wearable device or a sensor which is used by being connected to a wearable device.

According to this, for example, the burden on a user accompanying the detection of a stimulus can be reduced, and the sensor 100 can be favorably used, for example, even when an exercise is performed or the like. In addition, a user or the like can favorably confirm the detection result displayed on the display section. Further, such a configuration is preferred also from the viewpoint of fashion or the like.

Examples of the wearable device include a watch-type device.

In addition, for example, after the detection of a stimulus, the gel for a sensor 10 may be washed as needed. By doing this, the gel for a sensor 10 can be favorably reused, and the life of the gel for a sensor 10 and the sensor 100 can be prolonged. The washing of the gel for a sensor 10 can be performed using, for example, a liquid which does not contain a specific component (given stimulus).

When the sensor 100 is not used, the gel for a sensor 10 may be stored in a state where the gel for a sensor 10 is brought into contact with a liquid which does not contain a specific component (given stimulus). By doing this, the gel for a sensor 10 can be favorably stored, and the life of the gel for a sensor 10 and the sensor 100 can be prolonged.

Hereinabove, preferred embodiments have been described, however, the invention is not limited thereto.

For example, the sensor may have a configuration other than those described above.

For example, the sensor may include an adhesive layer for adhering the sensor to a given position.

In the above-mentioned embodiments, a case where the electrode is in contact with the gel for a sensor has been described, however, at least one intermediate layer may be provided between the electrode and the gel for a sensor. According to this, for example, the adhesiveness between the gel for a sensor and the electrode can be made more excellent.

Further, the sensor may have a partition that divides the gel for a sensor into a plurality of cells.

Further, the sensor may include a housing member that houses the gel for a sensor. According to this, for example, the gel for a sensor can be favorably protected from an external force or the like, and thus, the detection of a stimulus can be more stably performed.

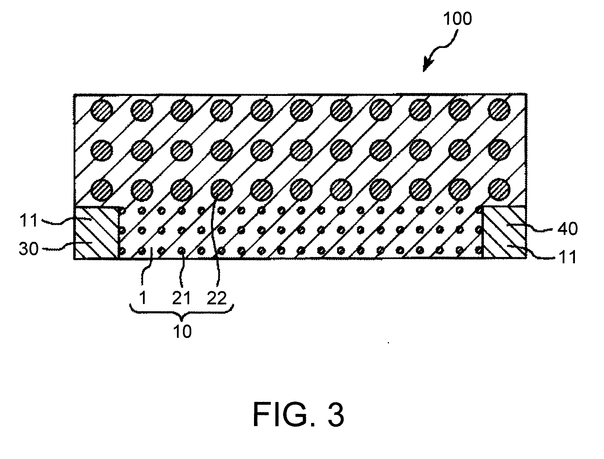

Further, in the above-mentioned embodiments, a case where the first particles and the second particles are both uniformly dispersed in the single stimulus-responsive gel has been representatively described, however, at least either of the first particles and the second particles may be unevenly distributed in a specific region in the stimulus-responsive gel. For example, the first particles may be unevenly distributed in a region including a line connecting the electrodes at the shortest distance (see FIG. 3). According to this, even in the case where the content of the first particles is reduced, the detection sensitivity can be made sufficiently excellent, and therefore, this configuration is advantageous from the viewpoint of suppression of the production cost of the gel for a sensor and the sensor (gel sensor). In addition, the first particles may be unevenly distributed in a region on a side (far side) away from the viewpoint of an observer in the stimulus-responsive gel or the second particles may be unevenly distributed in a region on a side near to the viewpoint of an observer in the stimulus-responsive gel (see FIG. 3. The upper side in FIG. 3 is defined as the viewpoint side of the observer.). According to this, the stimulus detection sensitivity and detection accuracy by an optical method can be made more excellent.

Further, in the above-mentioned embodiments, a case where the stimulus-responsive gel is in the form of a sheet and also the sensor as a whole is in the form of a sheet has been representatively described, however, the stimulus-responsive gel may be in any form other than a sheet, and also the sensor may be in any form, for example, in the form of a block, a string, a cylinder, or the like.

In addition, in the sensor, some constituent members may be configured to be replaceable or may be configured to be detachable and reattachable. For example, the absorbing member may be configured to be replaceable or detachable and reattachable. According to this, even if a large amount of a solid component (for example, a specific component or the like) is adhered to the absorbing member, by replacing the absorbing member, or detaching and washing the absorbing member, or the like, the sensor can be used. In this manner, the life of the sensor can be prolonged.

## Claims

1. A gel for a sensor, comprising:
a stimulus-responsive gel;
first particles which are contained in the stimulus-responsive gel and are constituted by a material containing an electrically conductive substance; and
second particles which are contained in the stimulus-responsive gel and contribute to structural coloration.

2. The gel for a sensor according to claim 1, wherein when the electrical resistivity of the electrically conductive substance is represented by R1 (Ω·m) and the electrical resistivity of the constituent material of the second particles is represented by R2 (Ω·m), the following relationship is satisfied: 1×10¹² ≤ R2/R1 ≤ 1×10²³.

3. The gel for a sensor according to claim 1 or 2, wherein the electrical resistivity of the electrically conductive substance is 1.0 ×10⁻³ Ω·m or less.

4. The gel for a sensor according to any one of the preceding claims, wherein the electrically conductive substance is constituted by a material containing one or more members selected from the group consisting of a metal material, an electrically conductive metal oxide, a carbon material, and an electrically conductive polymer material.

5. The gel for a sensor according to any one of the preceding claims 1, wherein when the refractive index of the constituent material of the first particles is represented by n1 and the refractive index of the constituent material of the second particles is represented by n2, the following relationship is satisfied: -0.8 ≤ n2-n1 ≤ 1.5.

6. The gel for a sensor according to any one of the preceding claims, wherein the average particle diameter of the second particles is 50 nm or more and 1000 nm or less.

7. The gel for a sensor according to any one of the preceding claims, wherein the content of the first particles with respect to 100 parts by volume of the stimulus-responsive gel when the stimulus-responsive gel is in an expanded state is 0.1 parts by volume or more and 50 parts by volume or less.

8. The gel for a sensor according to any one of the preceding claims, wherein the content of the second particles with respect to 100 parts by volume of the stimulus-responsive gel when the stimulus-responsive gel is in an expanded state is 0.1 parts by volume or more and 50 parts by volume or less.

9. The gel for a sensor according to any one of the preceding claims, wherein the gel for a sensor is provided with a recessed portion in a region coming into contact with an electrode.

10. The gel for a sensor according to claim 9, wherein in the recessed portion, the first particles are exposed on the surface.

11. A sensor, comprising:
the gel for a sensor according to any one of the preceding claims; and
an electrode.
